(19) Europäisches Patentamt
European Patent Office
Office européen des brevets

(11) EP 0 666 850 B1

# (12) FASCICULE DE BREVET EUROPEEN

(45) Date de publication et mention de la délivrance du brevet:
**05.02.1997 Bulletin 1997/06**

(21) Numéro de dépôt: **93923582.6**

(22) Date de dépôt: **18.10.1993**

(51) Int Cl.⁶: **C07D 217/04**, C07D 405/06, C07D 217/02, A61K 31/47

(86) Numéro de dépôt international:
**PCT/FR93/01022**

**WO 94/10150 (11.05.1994 Gazette 1994/11)**

(54) **DERIVES DE 1,2,3,5,6,7,8,8a-OCTAHYDRO-5,5,8a-TRIMETHYL-(8aBETA)-6-ISOQUINOLINEAMINE, LEUR PROCEDE DE PREPARATION ET LEUR UTILISATION EN THERAPEUTIQUE**

1,2,3,5,6,7,8,8a-OCTAHYDRO-5,5,8a-TRIMETHYL-(8aBETA)-6-ISOCHINOLINAMINE-DERIVATE, IHRE HERSTELLUNG UND VERWENDUNG ALS THERAPEUTIKA

1,2,3,5,6,7,8,8a-OCTAHYDRO-5,5,8a-TRIMETHYL-(8aBETA)-6-ISOQUINOLINEAMINE DERIVATIVES, PREPARATION METHOD THEREFOR AND THERAPEUTICAL USE THEREOF

(84) Etats contractants désignés:
**AT BE CH DE DK ES FR GB GR IE IT LI LU MC NL PT SE**

(30) Priorité: **28.10.1992 FR 9212865**

(43) Date de publication de la demande:
**16.08.1995 Bulletin 1995/33**

(73) Titulaire: **FOURNIER INDUSTRIE ET SANTE**
**F-75008 Paris (FR)**

(72) Inventeurs:
- **BINET, Jean**
  **F-21121 Fontaine-les-Dijon (FR)**
- **SAMRETH, Soth**
  **F-21600 Longvic (FR)**
- **DE FORNEL, Daniel**
  **F-21000 Dijon (FR)**
- **BOUCHER, Thierry**
  **F-21000 Dijon (FR)**
- **RENAUT, Patrice**
  **F-21121 Hauteville-les-Dijon (FR)**

(74) Mandataire: **Clisci, Serge et al**
**S.A. FEDIT-LORIOT & AUTRES**
**CONSEILS EN PROPRIETE INDUSTRIELLE**
**38, Avenue Hoche**
**75008 Paris (FR)**

(56) Documents cités:
**WO-A-89/08450** **US-A- 5 084 461**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Jouve, 75001 PARIS (FR)

EP 0 666 850 B1

## Description

La présente invention concerne des dérivés de 1,2,3,5,6,7,8,8a-octahydro-5,5,8a-triméthyl-(8aβ)-6-isoquinolineamine, inhibiteurs de la biosynthèse du cholestérol, notamment de l'époxysqualène cyclase, chez les mammifères et les champignons, leur procédé de préparation et leur utilisation en thérapeutique en tant qu'agents hypocholestérolémiants, hypolipémiants, antiathéromateux et antifongiques.

On connaît déjà divers composés inhibiteurs des différents enzymes intervenant dans la biosynthèse du cholestérol et notamment de la squalène époxydase et de l'époxysqualène cyclase.

De nombreuses études ont souligné l'importance et l'intérêt de ces produits dans la normalisation du taux de cholestérol et dans le domaine anti-fongique grâce à leur capacité à inhiber la biosynthèse d'ergostérol.

Par exemple, la demande de brevet EP-A-468434 décrit des éthers ou thioéthers de 4-hydroxypipéridine inhibiteurs de l'époxysqualène cyclase.

De même, les demandes de brevets EP-A-468457 et EP-A-420116 décrivent des dérivés de β-méthyl-4-pipéridineéthanol et des composés alkyl-4-pipéridinol en tant qu'inhibiteurs de la squalène époxydase et qui sont utiles en tant qu'agents antiathéromateux et antifongiques.

Parmi ces composés on trouve aussi des dérivés de décalines et d'azadécalines comme ceux décrits par exemple dans la demande internationale WO-A-89/08450 et le brevet US-A-5084461 utiles en tant qu'agents hypocholestérolémiants et antifongiques.

Aucun de ces documents antérieurs ne décrit ni ne suggère des dérivés de 1,2,3,5,6,7,8,8a-octahydro-5,5,8a-triméthyl-(8aβ)-6-isoquinolineamine, utiles comme inhibiteurs de la biosynthèse du cholestérol notamment de l'époxysqualène cyclase.

La présente invention propose donc des dérivés de 1,2,3,5,6,7,8,8a-octahydro-5,5,8a-triméthyl-(8aβ)-6-isoquinolineamine, inhibiteurs de la biosynthèse du cholestérol et notamment de l'époxysqualène cyclase.

La présente invention se rapporte aux composés choisis parmi l'ensemble constitué par les 1,2,3,5,6,7,8,8a-octahydro-5,5,8a-triméthyl-(8aβ)-6-isoquinolineamine, de formule :

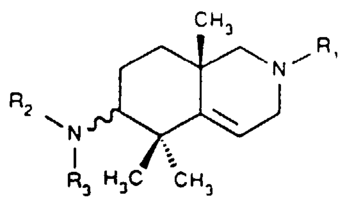

(I)

dans laquelle

- $R_1$ représente :
  - un groupe alkyle, linéaire ou ramifié, en $C_1$-$C_{12}$ éventuellement substitué par :
    * un groupe cycloalkyle,
    * un groupe oxirannyle,
    * un groupe phényle substitué par un groupe alkyle en $C_1$-$C_4$,
  - un groupe alkyle, linéaire ou ramifié, en $C_3$-$C_{12}$ comportant une ou plusieurs liaisons doubles ou triples, éventuellement substitué par un ou deux groupes phényle,
  - un groupe alkyle en $C_3$-$C_4$, linéaire ou ramifié, substitué par un ou plusieurs groupes hydroxyle,
- soit $R_2$ et $R_3$, identiques ou différents, représentent chacun un atome d'hydrogène ou un groupe alkyle en $C_1$-$C_4$, soit $R_2$ représente un atome d'hydrogène ou un groupe alkyle en $C_1$-$C_4$ et $R_3$ représente un groupe trifluoroacétyle ou un groupe acétyle,
- le symbole ∿ représente une liaison α ou β en position 6, et leurs sels d'addition.

Par sels d'addition, on vise ici les sels d'addition d'acide.

Par sels d'addition d'acide, on entend les sels obtenus avec les acides organiques, comme par exemple les acides 4-méthylbenzènesulfonique, (E)-2-butènedioïque, (Z)-2-butènedioïque, éthanedioïque, méthanesulfonique ou avec les acides minéraux, comme par exemple les acides chlorhydrique, bromhydrique, sulfurique, nitrique et phosphorique.

Par groupe alkyle en $C_1$-$C_{12}$ on entend ici un groupe alkyle, linéaire ou ramifié, comportant de 1 à 12 atomes de carbone.
Les groupes alkyle préférés sont les groupes n-propyle, méthyle, 2-méthyléthyle, 2-méthylpropyle et dodécyle.

Les groupes alkyle en $C_3$-$C_{12}$ comportant une ou plusieurs liaisons doubles ou triples préférés sont les groupes 2-propèn-1-yle, 6,6-diméthyl-hept-2-èn-4-yne-1-yle, 3-méthyl-2-butèn-1-yle, 3-phényl-2-propèn-1-yle, 3,3-diphényl-2-propèn-1-yle.

Parmi les groupes alkyle en $C_3$-$C_4$, linéaires ou ramifiés, comportant de 3 à 4 atomes de carbone et substitués par un ou plusieurs groupes hydroxyle le groupe 2,3-dihydroxypropyle est le groupe préféré.

Par groupe cycloalkyle, on entend ici un groupe alkyle cyclique comportant de 3 à 6 atomes de carbone ; le groupe cycloalkyle préféré selon l'invention est le cyclopropane.

Selon l'invention, on préfère les composés suivants :

**N-trifluoroacétyl-1,2,3,S,6,7,8,8a-octahydro-2-(2-propèn-1-yl)-5,5,8a-triméthyl-(6β,8aβ)-6-isoquinolineamine.**

**N,N-diméthyl-l,2,3,S,6,7,8,8a-octahydro-2-dodécyl-5,5,8a-triméthyl-(6β,8aβ)-6-isoquinolineamine, (E)-2-butènedioate.**

**N,N-diméthyl-1,2,3,5,6,7,8,8a-octahydro-2-(3-phényl-2-propèn-1-yl)-5,5,8a-triméthyl-(6β,8aβ)-6-isoquinolineamine, 4-méthylbenzènesulfonate.**

**N,N-diméthyl-1,2,3,5,6,7,8,8a-octahydro-2-[2-méthyl-3-[4-(1-méthyléthyl)phényl]propyl]-5,5,8a-triméthyl-(6β,8aβ)-6-isoquinolineamine, (E)-2-butènedioate.**

Les composés de formule I selon l'invention peuvent être préparés suivant un procédé caractérisé en ce que :

i) on soumet à une N-alkylation un composé de formule :

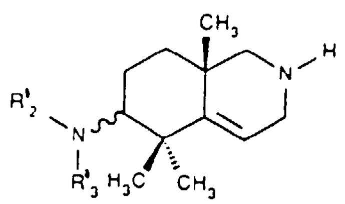

(II)

dans laquelle le symbole ~ représente une liaison α ou une liaison β en position 6 et, soit $R'_2$ et $R'_3$, identiques ou différents, représentent un groupe alkyle en $C_1$-$C_4$, soit $R'_2$ représente un atome d'hydrogène et $R'_3$ représente un groupe trifluoroacétyle par réaction avec un composé de formule $R'_1$-X dans laquelle X représente un atome d'halogène comme par exemple un atome de brome ou de chlore et $R'_1$ a la même définition que $R_1$ dans la formule I, en présence ou non d'un solvant polaire ou non polaire et aprotique, comme par exemple l'acétonitrile ou le N,N-diméthylformamide, en présence ou non d'un sel de métal alcalin, comme par exemple le carbonate de potassium, à raison de une mole de composé de formule II pour 1,1 à 1,2 moles de composé de formule $R'_1$-X à une température comprise entre la température ambiante et 150°C et pendant au moins une heure pour obtenir un composé de formule :

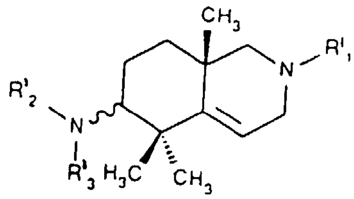

(I')

dans laquelle $R'_2$, $R'_3$ et $R'_1$ sont définis comme ci-dessus et le symbole ~ représente une liaison α ou une liaison β en position 6.

ii) si nécessaire on soumet les composés de formule I' ainsi obtenus à au moins un des traitements suivants :

a) les composés de formule I' dans laquelle $R'_1$ est défini comme précédemment, $R'_2$ représente un atome d'hydrogène, $R'_3$ représente un groupe trifluoroacétyle et le symbole ∿∿ représente une liaison α ou une liaison β en position 6 sont transformés, par alkylation de l'amide en position 6, selon les méthodes connues de l'homme de l'art, notamment en présence d'une base forte, comme par exemple l'hydrure de sodium, à raison de une mole de composé de formule I' pour une mole d'hydrure, à une température comprise entre 20 et 60°C durant au moins une heure, puis réaction avec un halogénure d'alkyle en $C_1$-$C_4$, comme par exemple l'iodure de méthyle, en présence d'un solvant approprié, comme par exemple le N,N-diméthylformamide, à raison de 1 mole de composé de formule I' pour 1,2 moles d'halogènure d'alkyle à une température proche de la température ambiante et durant quelques heures à plusieurs jours, en composés de formule I dans laquelle $R_1$ est défini comme précédemment, $R_2$ représente un groupe alkyle en $C_1$-$C_4$, $R_3$ représente un groupe trifluoroacétyle et le symbole ∿∿ représente une liaison α ou une liaison β en position 6 ;

b) on élimine le groupe trifluoroacétyle porté par l'amine en position 6 des composés de formule I' dans laquelle $R'_1$ a la même définition que précédemment, $R'_3$ représente un groupe trifluoroacétyle et $R'_2$ représente un atome d'hydrogène ou un groupe alkyle en $C_1$-$C_4$, par hydrolyse selon les méthodes connues de l'homme de l'art, notamment en présence d'un sel de métal alcalin, comme par exemple le carbonate de potassium, dans un alcool, comme par exemple le méthanol, en présence d'eau, à raison de une mole de composé I' pour un large excès de sel de métal alcalin, à une température comprise entre la température ambiante et 200°C et durant quelques heures à plusieurs jours, pour obtenir les composés de formule I dans laquelle $R_1$ a la même définition que précédemment, $R_3$ représente un atome d'hydrogène et $R_2$ représente un atome d'hydrogène ou un groupe alkyle en $C_1$-$C_4$ et le symbole ∿∿ représente une liaison α ou une liaison β en position 6 ;

c) on acyle les composés obtenus en b) selon les méthodes connues de l'homme de l'art, notamment par réaction avec un anhydride d'acide, comme par exemple l'anhydride acétique, dans un solvant approprié, comme par exemple le tétrahydrofuranne, en présence ou non de N,N-diéthyléthanamine, pour obtenir les composés de formule I dans laquelle $R_3$ représente un groupe acyle, $R_2$ représente un atome d'hydrogène ou un groupe alkyle en $C_1$-$C_4$, le symbole ∿∿ représente une liaison α ou une liaison β en position 6 et $R_1$ a la même définition que précédemment, à l'exception des composés de formule I où $R_1$ représente un groupe alkyle en $C_3$-$C_4$, linéaire ou ramifié, substitué par un ou plusieurs groupes hydroxyle ou encore un groupe alkyle, linéaire ou ramifié, en $C_1$-$C_{12}$ substitué par un groupe oxirannyle ;

d) les composés obtenus en b) dans lesquels $R_2$ et $R_3$ représentent tous deux un atome d'hydrogène sont transformés, par alkylation réductrice selon les méthodes connues de l'homme de l'art, notamment par réaction avec un composé approprié de formule $R_4$-CHO, dans laquelle $R_4$ représente un atome d'hydrogène ou un groupe alkyle en $C_1$-$C_3$, comme par exemple le formaldéhyde, puis ajustement du pH entre 6 et 8 et réduction de l'ion iminium formé, en présence d'un agent réducteur approprié, comme par exemple le cyanotriborohydrure de sodium, durant quelques heures à plusieurs jours, en composés de formule I dans laquelle $R_2$ et $R_3$, identiques ou différents, représentent chacun un groupe alkyle en $C_1$-$C_4$, $R_1$ a la même définition que précédemment et le symbole ∿∿ représente soit une liaison α soit une liaison β en position 6.

Suivant un autre procédé selon l'invention les composés de formule I' dans laquelle $R'_1$ a la même signification que précédemment et $R'_2$ et $R'_3$, identiques ou différents, représentent chacun un groupe alkyle en $C_1$-$C_4$, sont obtenus par une N-alkylation des composés de formule II dans laquelle $R'_2$ et $R'_3$, identiques ou différents, représentent un groupe alkyle en $C_1$-$C_4$ et le symbole ∿∿ une liaison α ou une liaison β en position 6, au moyen d'une amination réductrice par réaction avec un dérivé carbonylé de formule $R''_1$-CH=O, dans laquelle $R''_1$ est choisi de manière à conduire au groupe $R'_1$ souhaité par addition d'un carbone par rapport à $R''_1$ après réduction de l'ion iminium formé, selon les méthodes connues de l'homme de l'art, notamment en présence d'un agent réducteur approprié, comme par exemple le cyanotriborohydrure de sodium à température ambiante durant une heure à plusieurs jours, à raison de 1 mole de composé de formule II pour 1,1 à 1,2 moles de composé de formule $R''_1$-CH=O.

On peut aussi, suivant un autre procédé de synthèse selon l'invention, obtenir les composés de formule I' dans laquelle $R'_1$ a la même signification que précédemment, $R'_2$ et $R'_3$, identiques ou différents, représentent chacun un groupe alkyle en $C_1$-$C_4$ et le symbole ∿∿ représente une liaison α ou une liaison β en position 6, selon les méthodes connues de l'homme de l'art, notamment par une N-acylation des composés de formule II dans laquelle $R'_2$ et $R'_3$, identiques ou différents, représentent un groupe alkyle en $C_1$-$C_4$ et le symbole ∿∿ représente une liaison α ou une liaison β en position 6, avec un acide ou un dérivé d'acide approprié, comme par exemple un halogènure d'acide, suivie par une réduction en présence d'un agent réducteur, comme par exemple l'hydrure de bis (2-méthoxyéthoxy)

aluminium sodium, durant au moins une heure.

Pour accéder aux composés de formule II dans laquelle, soit R'$_2$ et R'$_3$, identiques ou différents, représentent chacun un groupe alkyle en $C_1$-$C_4$, soit R'$_2$ représente un atome d'hydrogène et R'$_3$ représente un groupe trifluoroacétyle , et le symbole ∿∿ représente une liaison α ou β en position 6, on préconise dans une première étape de préparer la 2-phénylméthyl-8a-méthyl-1,3,4,7,8,8a-hexahydro-6(2H)-isoquinolinone par condensation, selon les méthodes connues de l'homme de l'art, entre la 1-phénylméthyl-3-méthyl-4-pipéridone et la méthylvinylcétone, en présence d'une base forte, comme par exemple le méthanolate de sodium, puis de transformer cette dernière par réaction avec un large excès d'un composé de formule X-COO-$R_5$ dans laquelle X représente un atome d'halogène comme par exemple un atome de chlore et $R_5$ représente un groupe phénylméthyle ou un groupe alkyle en $C_1$-$C_4$, selon les méthodes connues de l'homme de l'art, notamment dans un solvant chloré, comme par exemple le trichlorométhane, à une température comprise entre 25 et 200°C durant plusieurs heures pour obtenir un composé de formule :

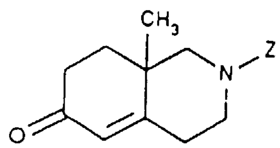

(III)

dans laquelle Z représente un groupe protecteur comme par exemple un groupe $COOR_5$ dans lequel $R_5$ représente un groupe phénylméthyle ou un groupe alkyle en $C_1$-$C_4$. On alkyle alors en position 5 les composés de formule III, selon les méthodes connues de l'homme de l'art, notamment par réaction avec un halogènure de méthyle, comme par exemple l'iodure de méthyle, dans un alcool, comme par exemple le 1,1-diméthyléthanol, en présence du sel de potassium du 1,1-diméthyléthanol, à une température comprise entre 20 et 150°C durant plusieurs heures pour obtenir les composés de formule :

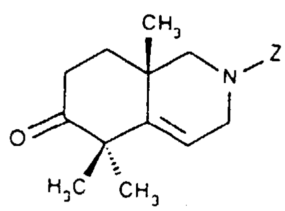

(IV)

dans laquelle Z a la même définition que précédemment. On réalise ensuite une amination réductrice du carbonyl en position 6 selon les méthodes connues de l'homme de l'art, notamment par réaction avec un dérivé aminé approprié sous forme de sel ou non, comme par exemple l'acétate d'ammonium, dans un alcool, comme par exemple le méthanol, et réduction par un agent réducteur approprié, comme par exemple le cyanotriborohydrure de sodium, à température ambiante durant quelques heures à plusieurs jours pour obtenir un composé de formule :

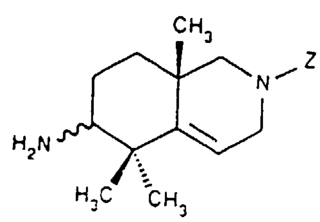

(V)

dans laquelle Z a la même signification que précédemment.

On acyle ensuite les composés de formule V selon les méthodes connues de l'homme de l'art, notamment par réaction avec l'anhydride trifluoroacétique dans un solvant approprié, comme par exemple le tétrahydrofuranne, en présence ou non de N,N-diméthyléthanamine pour obtenir les composés de formule :

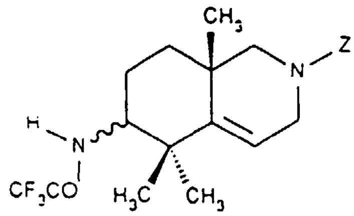

(VI)

dans laquelle Z a la même définition que précédemment et l'on sépare les isomères 6α et 6β selon les méthodes connues de l'homme de l'art, notamment par chromatographie sur colonne de silice.
On obtient alors directement les composés de formule II, dans laquelle $R'_2$ représente un atome d'hydrogène et $R'_3$ représente un groupe trifluoroacétyle, sous forme d'isomère α ou β par déprotection de l'amine cyclique de formule VI, dans laquelle Z représente un groupe $COOR_5$ dans lequel $R_5$ représente un groupe phénylméthyle, selon les méthodes connues de l'homme de l'art, notamment par hydrogénolyse sous atmosphère d'hydrogène, en présence d'un catalyseur, comme par exemple du charbon palladié, dans un alcool, comme par exemple l'éthanol.

Les composés de formule II dans laquelle $R'_2$ ou $R'_3$, identiques ou différents, représentent un groupe alkyle en $C_1$-$C_4$, sont obtenus de la façon suivante :

Dans une première étape, on hydrolyse le groupe trifluoroacétyle des isomères α ou β des composés de formule VI dans laquelle Z a la même définition que précédemment, selon les méthodes connues de l'homme de l'art, notamment par réaction avec un sel de métal alcalin, comme par exemple le carbonate de potassium, dans un alcool, en présence d'eau, pour obtenir les composés de formule :

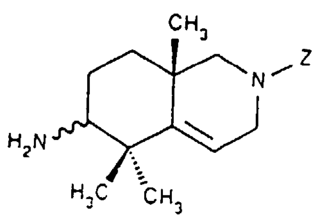

(V')

dans laquelle Z représente un groupe protecteur, comme par exemple un groupe $COOR_5$ dans lequel $R_5$ représente un groupe phénylméthyle ou un groupe alkyle en $C_1$-$C_4$ et le symbole ∿∿ représente une liaison α ou une liaison β en position 6.

Dans une deuxième étape, on transforme les composés de formule V', au moyen d'une alkylation réductrice, selon les méthodes connues de l'homme de l'art, notamment par réaction avec un dérivé carbonylé approprié, comme par exemple le formaldéhyde, puis réduction avec un agent réducteur approprié, comme par exemple le cyanotriborohydrure de sodium, pour obtenir les composés de formule :

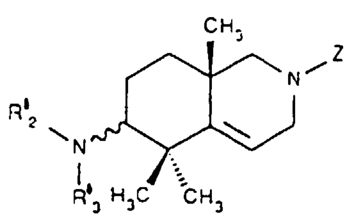

(VII)

dans laquelle Z est défini comme précédemment, le symbole ∿∿ représente une liaison α ou une liaison β en position 6 et $R'_2$ et $R'_3$, identiques ou différents, représentent chacun un groupe alkyle en $C_1$-$C_4$.

Enfin, dans une troisième étape, on déprotège, par élimination du groupe Z, les composés de formule VII, selon les méthodes connues de l'homme de l'art, notamment par hydrogénolyse en présence d'un catalyseur, comme par exemple le charbon palladié, si Z représente un groupe $COOR_5$ dans lequel $R_5$ représente un groupe phénylméthyle ou par réaction avec un dérivé silylé, comme par exemple le chlorure de triméthylsilyle, en présence d'iodure de sodium pendant plusieurs heures à une température comprise entre 20° et 200°C si Z représente un groupe $COOR_5$ dans

lequel $R_5$ représente un groupe alkyle en $C_1$-$C_4$, pour obtenir les composés de formule II recherchés.

Les composés intermédiaires de formule II dans laquelle le symbole ∿ représente une liaison $\alpha$ ou une liaison $\beta$ en position 6 et, soit $R'_2$ et $R'_3$, identiques ou différents, représentent chacun un groupe alkyle en $C_1$-$C_4$, soit $R'_2$ représente un atome d'hydrogène et $R'_3$ représente un groupe trifluoroacétyle sont des composés nouveaux et constituent un des objets de l'invention.

Les composés intermédiaires de formule VI dans laquelle le symbole ∿ représente une liaison $\alpha$ ou une liaison $\beta$ en position 6 et Z représente un groupe protecteur, comme par exemple un groupe $COOR_5$ dans lequel $R_5$ représente un groupe phénylméthyle ou un groupe alkyle en $C_1$-$C_4$, sont des composés nouveaux et constituent un des objets de l'invention.

Les composés intermédiaires de formule III, IV, V et V' dans lesquelles Z représente un groupe protecteur, comme par exemple un groupe $COOR_5$ dans lequel $R_5$ représente un groupe phénylméthyle ou un groupe alkyle en $C_1$-$C_4$, sont des composés nouveaux.

Les composés intermédiaires de formule VII dans laquelle le symbole ∿ représente une liaison $\alpha$ ou une liaison $\beta$ en position 6, Z représente un groupe protecteur, comme par exemple un groupe $COOR_5$ dans lequel $R_5$ représente un groupe phénylméthyle ou un groupe alkyle en $C_1$-$C_4$, et $R'_2$ et $R'_3$, identiques ou différents, représentent chacun un groupe alkyle en $C_1$-$C_4$ sont des composés nouveaux.

L'invention sera mieux comprise à la lecture des exemples de préparation qui suivent. Ces exemples sont destinés à illustrer l'invention sans en limiter la portée. Par commodité, dans ce qui suit les "Préparations" se réfèrent à l'obtention des précurseurs et intermédiaires, et les "Exemples" se réfèrent à l'obtention des produits de formule I selon l'invention.

**PREPARATION I**

**2-phénylméthyl-8a-méthyl-1,3,4,7,8,8a-hexahydro-6(2H)-isoquinolinone**

On ajoute 101,5 g ($5.10^{-1}$ mole) de 1-phénylméthyl-3-méthyl-4-pipéridone à une solution de méthanolate de sodium préalablement préparée à partir de 12,7 g ($5{,}5.10^{-1}$ mole) de sodium dans un litre de méthanol. On agite 45 minutes à température ambiante, refroidit la solution à 5°C et additionne goutte à goutte en 2 heures environ 62,3 ml ($7{,}5.10^{-1}$ mole) de méthylvinylcétone.

On laisse le mélange revenir à 20°C et reposer toute une nuit. On ajoute 55 ml d'acide chlorhydrique concentré et évapore à sec. On reprend le résidu avec du 1,1'-oxybis-éthane et de l'eau. La phase organique est lavée à l'eau, séchée avec du sulfate de magnésium, filtrée et les solvants sont évaporés sous pression réduite.

On purifie l'huile obtenue par chromatographie sur colonne de silice en éluant au moyen du mélange dichlorométhane/ 2,2'-oxybis-propane 9/1 (v/v) pour récupérer 37,8 g (rendement : 30 %) du produit recherché qui cristallise dans le n-pentane.

F = 98°C

**PREPARATION II**

**Phénylméthyl ester de l'acide 2,3,1,6,7,8-hexahydro-8a-méthyl-6-oxo-2(1H)-isoquinolinecarboxylique**

On porte à reflux pendant une nuit un mélange de 35 g ($1{,}4.10^{-1}$ mole) de 2-(phénylméthyl)-8a-méthyl-1,3,4,7,8,8a-hexahydro-6(2H)-isoquinolinone et de 99 ml ($7.10^{-2}$ mole) du phénylméthyl ester de l'acide chloroformique en présence de 28,6 g ($2.10^{-2}$ mole) de carbonate de potassium dans 300 ml de trichlorométhane. On refroidit le mélange, filtre, lave le filtrat avec de l'eau, sèche et évapore les solvants sous pression réduite.

On triture l'huile résiduelle récupérée dans l'éther de pétrole. On obtient 36 g (rendement : 87 %) du produit recherché.
F = 128°C

En procédant selon le même mode opératoire mais à partir de l'ester éthylique de l'acide chloroformique, on obtient le produit suivant :

**Ester éthylique de l'acide 2,3,4,6,7,8-hexahydro-8a-méthyl-6-oxo-2(1H)-isoquinolinecarboxylique**
F = 66-69°C

**PREPARATION III**

**Phénylméthyl ester de l'acide 3,5,6,7,8,8a-hexahydro-5,5,8a-triméthyl-6-oxo-(8aβ)-2(1H) -isoquinolinecarboxylique.**

On solubilise 35 g (1,2.$10^{-1}$ mole) du phénylméthyl ester de l'acide 2,3,4,6,7,8-hexahydro-8a-méthyl-6-oxo-2(1H)-isoquinolinecarboxylique dans 350 ml de 1,1-diméthyléthanol. On additionne alors en une seule fois 39,4 g (3,5.$10^{-1}$ mole) du sel de potassium du 1,1-diméthyléthanol.
Le mélange se colore en rouge. On agite 1 heure à 50°C et ajoute avec précaution, goutte à goutte, en 30 minutes 44,3 ml (7.$10^{-1}$ mole) d'iodométhane en solution dans le 1,1-diméthyléthanol. Un précipité jaune apparaît. A la fin de l'addition, on chauffe le mélange à 50°C pendant 1 heure puis on agite une nuit à température ambiante. On verse le mélange dans de l'eau et extrait à l'ester éthylique de l'acide acétique. La phase organique est lavée à l'eau, séchée avec du sulfate de magnésium, filtrée et les solvants sont évaporés sous pression réduite.
Après purification par chromatographie sur colonne de silice en éluant au moyen du mélange méthylbenzène/ester éthylique de l'acide acétique 9/1 (v/v) on récupère 23,6 g (rendement : 62 %) du produit recherché sous forme d'huile légèrement jaune.
$n^{31}_D$ = 1,5375

En procédant de manière analogue, à partir de l'ester éthylique de l'acide 2,3,4,6,7,8-hexahydro-8a-méthyl-6-oxo-2(1H)-isoquinolinecarboxylique, on obtient le produit suivant :
**Ester éthylique de l'acide 3,5,6,7,8,8a-hexahydro-5,5,8a-triméthyl-6-oxo-(8aβ)-2(1H)-isoquinolinecarboxylique.**
F = 58°C

**PREPARATION IV**

**Phénylméthyl ester de l'acide 3,5,6,7,8,8a-hexabydro-5,5,8a-triméthyl-6-amino-(8aβ)-2(1H) -isoquinolinecarboxylique.**

On mélange une solution de 65 g (2.$10^{-1}$ mole) du phénylméthyl ester de l'acide 3,5,6,7,8,8a-hexahydro-5,5,8a-triméthyl-6-oxo-(8aβ)-2(1H)-isoquinolinecarboxylique dans 700 ml de méthanol avec 153 g (2 moles) d'acétate d'ammonium et on ajuste le pH de la solution à 7,3 par addition d'acide acétique, puis on ajoute par portions 20 g (3.$10^{-1}$ mole) de cyanotriborohydrure de sodium.
On agite la solution pendant 2 jours à température ambiante. On évapore le méthanol, on reprend le résidu d'évaporation avec une solution d'hydroxyde de sodium et on extrait à l'ester éthylique de l'acide acétique. La phase organique est lavée à l'eau, séchée avec du sulfate de magnésium, filtrée et les solvants sont évaporés sous pression réduite.
On solubilise l'huile récupérée dans de l'acide chlorhydrique 5N et on extrait à l'aide de 1,1'-oxybiséthane.
La phase aqueuse est alcalinisée avec une solution d'hydroxyde de sodium et on extrait avec l'ester éthylique de l'acide acétique.
La phase organique est lavée à l'eau, séchée avec du sulfate de magnésium, filtrée et les solvants sont évaporés sous pression réduite.
On obtient 52 g (rendement : 80 %) du produit recherché sous forme d'huile jaune.
$n^{34}_D$ = 1,5469

De façon analogue, à partir de l'ester éthylique de l'acide 3,5,6,7,8,8a-hexahydro-5,5,8a-triméthyl-6-oxo-(8aβ)-2 (1H)-isoquinolinecarboxylique, on obtient le produit suivant :
**Ester éthylique de l'acide 3,5,6,7,8,8a-hexahydro-5,5,8a-triméthyl-6-amino-(8aβ)-2(1H)-isoquinolinecarboxylique, (E)-2-butènedioate.**
F = 140-145°C

**PREPARATION V**

**Phénylméthyl ester de l'acide 3,5,6,7,8,8a-hexahydro-5,5,8a-triméthyl-6-(trifluoroacétylamino)-(8aβ)-2(1H) -isoquinolinecarboxylique.**

On solubilise 50 g (1,5.$10^{-1}$ mole) du phénylméthyl ester de l'acide 3,5,6,7,8,8a-hexahydro-5,5,8a-triméthyl-6-amino-(8aβ)-2(1H)-isoquinolinecarboxylique dans 200 ml de tétrahydrofuranne et 25,5 ml de N,N-diéthyléthanamine. On refroidit la solution à 0°C environ et additionne goutte à goutte 25,8 ml (1,8.$10^{-1}$ mole) d'anhydride trifluoroacétique en solution dans 50 ml de tétrahydrofuranne.

On agite une nuit à température ambiante. On évapore le mélange réactionnel sous pression réduite et reprend l'huile résiduelle avec du 1,1'-oxybis-éthane. La phase organique est lavée avec une solution d'acide chlorhydrique

1N puis avec de l'eau, séchée avec du sulfate de magnésium, filtrée et les solvants sont évaporés sous pression réduite. On obtient ainsi le composé recherché sous forme d'huile.
Après purification de l'huile obtenue par chromatographie sur colonne de silice en éluant au moyen du mélange 2,2'-oxybis-propane/méthylcyclohexane 9/1 (v/v), on récupère 6,5 g (rendement : 12 %) du produit suivant :
Phénylméthyl ester de l'acide 3,5,6,7,8,8a-hexahydro-5,5,8a-triméthyl-6-(trifluoroacétylamino)-(6α,8aβ)-2(1H)-isoquinolinecarboxylique.
$n^{36}_D$ = 1,5145
et 37,1 g (rendement : 59 %) du produit suivant :
**Phénylméthyl ester de l'acide 3,5,6,7,8,8a-hexahydro-5,5,8a-triméthyl-6-(trifluoroacétylamino)-(6β,8aβ)-2(1H)-isoquinolinecarboxylique.**
F = 138°C

De façon analogue à partir de l'ester éthylique de l'acide 3,5,6,7,8,8a-hexahydro-5,5,8a-triméthyl-6-amino-(8aβ)-2(1H)-isoquinolinecarboxylique, on obtient après purification les 2 isomères suivants :
**Ester éthylique de l'acide 3,5,6,7,8,8a-hexahydro-5,5,8a-triméthyl-6-(trifluoroacétylamino)-(6α,8aβ)-2(1H)-isoquinolinecarboxylique.**
$n^{32}_D$ = 1,4845
**Ester éthylique de l'acide 3,5,6,7,8,8a-hexahydro-5,5,8a-triméthyl-6-(trifluoroacétylamino)-(6β,8aβ)-2(1H)-isoquinolinecarboxylique.**
F = 70-80°C

## PREPARATION VI

### N-trifluoroacétyl-1,2,3,5,6,7,8,8a-octahydro-5,5,8a-triméthyl-(6β,8aβ)-6-isoquinolineamine, 4-méthylbenzènesulfonate

On hydrogène à température ambiante, sous pression atmosphérique, 27 g ($6.10^{-2}$ mole) de phénylméthyl ester de l'acide 3,5,6,7,8,8a-hexahydro-5,5,8a-triméthyl-6-(trifluoroacétylamino)-(6β,8aβ)-2(1H)-isoquinoline--carboxylique en solution dans 150 ml d'éthanol et en présence de 2,7 g de charbon palladié à 5 %. Lorsque la réaction est terminée, on filtre le mélange réactionnel, élimine le catalyseur et évapore le filtrat à sec.
Après trituration du résidu récupéré dans le 2,2'-oxybis-propane, on obtient 16,5 g (rendement : 90 %) de produit fondant à 154°C à partir duquel on prépare dans l'éthanol le 4-méthylbenzènesulfonate.
F > 260°C

De façon analogue à partir de l'isomère (6α,8aβ) du phénylméthyl ester de l'acide 3,5,6,7,8,8a-hexahydro-5,5,8a-triméthyl-6-(trifluoroacétylamino)-2(1H)-isoquinolinecarboxylique, on prépare le produit suivant :
**N-trifluoroacétyl-1,2,3,5,6,7,8,8a-octahydro-5,5,8a-triméthyl-(6α,8aβ)-isoquinolineamine**
$n^{31}_D$ = 1,4902

## Exemple 1

**N-trifluoroacétyl-1,2,3,5,6,7,8,8a-octahydro-2-(2-propèn-1-yl)-5,5,8a-triméthyl-(6β,8aβ)-6-isoquinolineamine.**

A un mélange de 2 g ($7.10^{-3}$ mole) de N-trifluoroacétyl-1,2,3,5,6,7,8,8a-octahydro-5,5,8a-triméthyl-(6β,8aβ)-6-isoquinolineamine et 1,9 g de carbonate de potassium anhydre dans 60 ml d'acétonitrile on ajoute goutte à goutte une solution de 0,7 ml ($8.10^{-3}$ mole) de 3-bromo-1,2-propène dans 2 ml d'acétonitrile.
Après chauffage à reflux durant une heure, on filtre le précipité, évapore le filtrat à sec et reprend l'huile résiduelle avec de l'eau. On extrait au 1,1'-oxybis-éthane. La phase organique est séchée avec du sulfate de magnésium, filtrée et les solvants sont évaporés sous pression réduite.
L'huile ainsi récupérée est purifiée par chromatographie sur colonne de silice en éluant au moyen du mélange dichlorométhane/méthanol 98/2 (v/v). On obtient 2 g (rendement : 91 %) de produit blanc.
F = 130°C

En procédant de façon analogue à la synthèse précédente, on prépare les composés suivants :

## Exemple 2

**N-trifluoroacétyl-1,2,3,S,6,7,8,8a-octahydro-2-propyl-5,5,8a-triméthyl-(6β,8aβ)-6-isoquinolineamine.**
F = 105-108°C

**Exemple 3**

**N-trifluoroacétyl-1,2,3,S,6,7,8,8a-octahydro-2-(cyclopropylméthyl)-5,5,8a-triméthyl-(6β,8aβ)-6-isoquinolineamine, (E)-2-butènedioate.**
F = 193-197°C

**Exemple 4**

**N-trifluoroacétyl-1,2,3,5,6,7,8,8a-octahydro-2-(6,6-diméthyl-2-heptèn-4-yn-1-yl)-5,5,8a-triméthyl-(6β,8aβ)-6-isoquinolineamine.**
F = 131°C

**Exemple 5**

**N-trifluoroacétyl-1,2,3,5,6,7,8,8a-octahydro-2-[1-(2,3 époxypropyl)]-5,5,8a-triméthyl-(6β,8aβ)-6-isoquinolineamine.**
F = 130°C

**Exemple 6**

**N-trifluoroacétyl-1,2,3,5,6,7,8,8a-octahydro-2-[1-(2,3-dihydroxypropyl)]-5,5,8a-triméthyl-(6β,8aβ)-6-isoquinolineamine, E-2-butènedioate.**
F = 186°C

**Exemple 7**

**N-trifluoroacétyl-1,2,3,5,6,7,8,8a-octahydro-2-(3-méthyl-2-butèn-1-yl)-5,5,8a-triméthyl-(6β,8aβ)-6-isoquinolineamine, éthanedioate.**
F = 230°C

**Exemple 8**

**N-trifluoroacétyl-1,2,3,5,6,7,8,8a-octahydro-2-dodécyl-5,5,8a-triméthyl-(6β,8aβ)-6-isoquinolineamine.**
F = 80°C

**Exemple 9**

**N-trifluoroacétyl-1,2,3,5,6,7,8,8a-octahydro-2-(2-propèn-1-yl)-5,5,8a-triméthyl-(6α,8aβ)-6-isoquinolineamine.**
F = 76°C

**Exemple 10**

**N-méthyl-N-trifluoroacétyl-1,2,3,5,6,7,8,8a-octahydro-2-dodécyl-5,5,8a-triméthyl-(6β,8aβ)-6-isoquinolineamine.**

A une suspension de 0,43 g ($1,3.10^{-2}$ mole) d'hydrure de sodium à 60 % dans 10 ml de N,N-diméthylformamide, on ajoute 3,5 g ($1,1.10^{-2}$ mole) de N-trifluoroacétyl-1,2,3,5,6,7,8,8a-octahydro-2-dodécyl-5,5,8a-triméthyl-(6β,8aβ)-6-isoquinolineamine en solution dans 20 ml de N,N-diméthylformamide.
A la fin de l'addition on chauffe le mélange à 40°c durant une heure. On refroidit, ajoute 0,8 ml d'iodométhane ($1,3.10^{-2}$ mole) et agite durant deux jours à température ambiante. On verse le mélange réactionnel dans de l'eau et on extrait avec le 1,1'-oxybis-éthane. La phase organique est lavée à l'eau, séchée avec du sulfate de magnésium, filtrée et les solvants sont évaporés sous pression réduite. On obtient 1,7 g (rendement : 47 %) du produit recherché.
F = 132°C

En procédant de façon analogue à la synthèse précédente, on prépare le composé suivant :

**Exemple 11**

**N-méthyl-N-trifluoracétyl-1,2,3,5,6,7,8,8a-octahydro-2-(2-propèn-1-yl)-5,5,8a-triméthyl-(6β,8aβ)-6-isoqui-**

**nolineamine.**
F = 80°C

**Exemple 12**

**N-méthyl-1,2,3,5,6,7,8,8a-octahydro-2-dodécyl-5,5,8a-triméthyl-(6β,8aβ)-6-isoquinolineamine, éthanedioate.**

On porte à reflux pendant 48 heures un mélange de 2,5 g ($5.10^{-3}$ mole) de N-trifluoroacétyl-N-méthyl-1,2,3,5,6,7,8,8a-octahydro-2-dodécyl-5,5,8a-triméthyl-(6β,8aβ)-6-isoquinolineamine et de 14,6 g ($10.10^{-2}$ mole) de carbonate de potassium dans 200 ml de méthanol et 50 ml d'eau.
On évapore le méthanol, reprend le résidu d'évaporation avec de l'eau et on extrait avec du trichlorométhane. La phase organique est lavée à l'eau, séchée avec du sulfate de magnésium, filtrée et on évapore les solvants sous pression réduite.
On récupère une huile que l'on purifie par chromatographie sur colonne de silice en éluant au moyen du mélange dichlorométhane/méthanol/ammoniaque 98,5/1/0,5 (v/v/v).
On obtient 1,5 g (rendement = 79 %) d'huile à partir de laquelle on prépare l'éthanedioate recherché.
F = 134°C

De façon analogue à la synthèse précédente, on prépare les composés suivants :

**Exemple 13**

**N-méthyl-1,2,3,5,6,7,8,8a-octahydro-2-(2-propèn-1-yl)-5,5,8a-triméthyl-(6β,8aβ)-6-isoquinolineamine, éthanedioate.**
F = 180°C

**Exemple 14**

**1,2,3,5,6,7,8,8a-octahydro-2-dodécyl-5,5,8a-triméthyl-(6β,8aβ)-6-isoquinolineamine, E-2-butènedioate.**
F = 155°C

Ce produit est obtenu à partir du N-trifluoroacétyl-1,2,3,5,6,7,8,8a-octahydro-2-dodécyl-5,5,8a-triméthyl-(6β,8aβ)-6-isoquinolineamine.

**Exemple 15**

**1,2,3,5,6,7,8,8a-octahydro-2-(2-propèn-1-yl)-5,5,8a-triméthyl-(6β,8aβ)-6-isoquinolineamine, 4-méthylbenzènesulfonate.**
F = 148°C

Ce produit est obtenu à partir du N-trifluoroacétyl-1,2,3,5,6,7,8,8a-octahydro-2-(2-propèn-1-yl)-5,5,8a-triméthyl-(6β,8aβ)-6-isoquinolineamine.

**Exemple 16**

**N-acétyl-1,2,3,5,6,7,8,8a-octahydro-2-(2-propèn-1-yl)-5,5,8a-triméthyl-(6β,8aβ)-6-isoquinolineamine, 4-méthylbenzènesulfonate.**

A une solution de 1,5 g ($6,4.10^{-3}$ mole) de 1,2,3,5,6,7,8,8a-octahydro-2-(2-propèn-1-yl)-5,5,8a-triméthyl-(6β,8aβ)-6-isoquinolineamine dans 20 ml de tétrahydrofuranne et de 1,1 ml de N,N-diéthyléthanamine on ajoute goutte à goutte 0,73 ml ($7,8.10^{-3}$ mole) d'anhydride acétique dilué dans 5 ml de tétrahydrofuranne.

On agite le mélange réactionnel pendant 4 heures puis on le verse dans de l'eau et on extrait avec du 1,1'-oxybiséthane. La phase organique est lavée à l'eau, séchée avec du sulfate de magnésium, filtrée et on évapore les solvants sous pression réduite. On purifie l'huile obtenue par chromatographie sur une colonne de silice en éluant au moyen du mélange dichlorométhane/méthanol 95/5 (v/v).
Après évaporation des solvants des fractions purifiées, on récupère 1 g d'huile à partir de laquelle on prépare le 4-méthylbenzènesulfonate dans la 2-propanone. Par recristallisation dans un mélange butanone/2-propanol 98/2 (v/v) on cbtient 1 g (rendement : 35 %) du produit recherché.
F = 224°C

**PREPARATION VII**

**Phénylméthyl ester de l'acide 3,5,6,7,8,8a-hexahydro-5,5,8a-triméthyl-6-amino-(6β,8aβ)-2(1H) -isoquinolinecarboxylique.**

On chauffe à reflux pendant 8 heures un mélange de 35 g ($8.10^{-2}$ mole) de phénylméthyl ester de l'acide 3,5,6,7,8,8a-hexahydro-5,5,8a-triméthyl-6-(trifluoroacétylamino)-(6β,8aβ)-2(1H)-isoquinoline--carboxylique dans 500 ml de méthanol et 100 ml d'eau avec 115 g ($8.10^{-1}$ mole) de carbonate de potassium.
Après évaporation du mélange réactionnel, on reprend le résidu récupéré avec de l'eau et on extrait à l'ester éthylique de l'acide acétique. La phase organique est lavée à l'eau, séchée avec du sulfate de magnésium, filtrée et les solvants sont évaporés sous pression réduite. On obtient 27 g (rendement : 100 %) du produit recherché.
$n^{24,5}_D$ = 1,5523

De la même façon à partir de l'isomère (6α,8aβ) du phénylméthyl ester de l'acide 3,5,6,7,8,8a-hexahydro-5,5,8a-triméthyl-6-(trifluoroacétylamino)-2(1H)-isoquinolinecarboxylique, on obtient le produit suivant :
**Phénylméthyl ester de l'acide 3,5,6,7,8,8a-hexahydro-5,5,8a-triméthyl-6-amino-(6α,8aβ)-2(1H)-isoquinolinecarboxylique.**
$n^{28}_D$ = 1,5121

**PREPARATION VIII**

**Phénylméthyl ester de l'acide 3,5,6,7,8,8a-hexahydro-5,5,8a-triméthyl-6-diméthylamino-(6β,8aβ)-2(1H) -isoquinolinecarboxylique.**

On mélange 27 g ($8.10^{-2}$ mole) du phénylméthyl ester de l'acide 3,5,6,7,8,8a-hexahydro-5,5,8a-triméthyl-6-amino-(6β,8aβ)-2(1H)-isoquinolinecarboxylique dans 500 ml d'acétonitrile avec 74 ml de formaldéhyde en solution aqueuse à 37 %. On agite 10 minutes à environ zéro degré et ajuste le pH à 7,5 par addition d'acide acétique. On ajoute alors par fractions 15,5 g ($2,5.10^{-1}$ mole) de cyanotriborohydrure de sodium et on agite 12 heures à température ambiante.
Après évaporation du mélange réactionnel on reprend le résidu obtenu avec une solution d'hydroxyde de sodium et on extrait à l'ester éthylique de l'acide acétique. La phase organique est lavée à l'eau, séchée avec du sulfate de magnésium, filtrée et les solvants sont évaporés sous pression réduite.
Après purification de l'huile résiduelle récupérée par chromatographie sur colonne de silice en éluant au moyen du mélange dichlorométhane/méthanol/ammoniaque 8,5/1/0,5 (v/v/v), on obtient 23,5 g (rendement : 80 %) d'huile que l'on utilise brute.
$n^{24,5}_D$ = 1,5358

De façon analogue à la synthèse précédente, on prépare les composés suivants :
**Ester éthylique de l'acide 3,5,6,7,8,8a-hexahydro-5,5,8a-triméthyl-6-diméthylamino-(6α,8aβ)-2(1H)-isoquinolinecarboxylique.**
$n^{29}_D$ = 1,5051
**Ester éthylique de l'acide 3,5,6,7,8,8a-hexahydro-5,5,8a-triméthyl-6-diméthylamino-(6β,8aβ)-2(1H)-isoquinolinecarboxylique.**
F = 54°C

De même à partir des isomères (6β,8aβ) (Exemple 8) et (6α,8aβ) du 1,2,3,5,6,7,8,8a-octahydro-2-dodécyl-5,5,8a-triméthyl-6-isoquinolineamine, on prépare les composés suivants :

**Exemple 17**

**N,N-diméthyl-1,2,3,5,6,7,8,8a-octahydro-2-dodécyl-5,5,8a-triméthyl-(6β,8aβ)-6-isoquinolineamine, (E)-2-butènedioate.**
F = 180°C

**Exemple 18**

**N,N-diméthyl-1,2,3,S,6,7,8,8a-octahydro-2-dodécyl-5,5,8a-triméthyl-(6α,8aβ)-6-isoquinolineamine, éthanedioate.**
F = 121-129°C

**PREPARATION IX**

**N,N-diméthyl-1,2,3,5,6,7,8,8a-octahydro-5,5,8a-triméthyl-(6α,8aβ)-6-isoquinolineamine.**

On solubilise 0,8 g (2,7.$10^{-3}$ mole) d'ester éthylique de l'acide 3,5,6,7,8,8a-hexahydro-5,5,8a-triméthyl-6-diméthylamino-(6α,8aβ)-2(1H)-isoquinolinecarboxylique dans 15 ml d'acétonitrile et 2,6 g de iodure de sodium. On ajoute lentement 2 ml de chlorure de triméthylsilyle et on porte le mélange réactionnel à reflux durant une nuit. On ajoute à nouveau 2,6 g de iodure de sodium puis 2 ml de chlorure de triméthylsilyle et porte à nouveau à reflux durant 24 heures. Après évaporation sous pression réduite, on reprend le résidu par une solution d'acide chlorhydrique 1N et on extrait au 1,1'-oxybis-éthane. On alcalinise la phase aqueuse avec une solution d'hydroxyde de sodium et extrait au 1,1'-oxybis-éthane. La phase organique est lavée à l'eau, séchée avec du sulfate de magnésium, filtrée et les solvants sont évaporés sous pression réduite. On obtient 0,5 g (rendement : 77 %) du produit recherché.
F = 103°C

**PREPARATION X**

**N,N-diméthyl-1,2,3,5,6,7,8,8a-octahydro-5,5,8a-triméthyl-(6β,8aβ)-6-isoquinolineamine.**

On hydrogène à température ambiante et sous pression atmosphérique une solution de 21,5 g (6.$10^{-2}$ mole) de phénylméthyl ester de l'acide 3,5,6,7,8,8a-hexahydro-6-diméthylamino-5,5,8a-triméthyl-(6β,8aβ)-2(1H)-isoquinolinecarboxylique dans 250 ml d'acide acétique en présence de 3,5 g de charbon palladié à 5 %. Lorsque la réaction est terminée, on élimine le catalyseur par filtration et on évapore le filtrat à sec. On reprend l'huile résiduelle obtenue avec de l'eau, alcalinise avec une solution d'hydroxyde de sodium et on extrait au 1,1'-oxybis-éthane.
La phase organique est lavée à l'eau, séchée avec du sulfate de magnésium, filtrée et on évapore les solvants sous pression réduite.
On récupère 12,6 g (rendement : 95 %) du produit recherché.
F = 92°C

**Exemple 19**

**N,N-diméthyl-1,2,3,5,6,7,8,8a-octahydro-2-(6,6-diméthyl-2-heptèn-4-yne-1yl)-5,5,8a-triméthyl-(6β,8aβ)-6-isoquinolineamine.**

On ajoute goutte à goutte une solution de 3,8 g (1,9.$10^{-2}$ mole) de 1-bromo-6,6-diméthyl-2-heptèn-4-yne (E/Z = 3/1) dans 20 ml d'acétonitrile à un mélange de 3,5 g (1,6.$10^{-2}$ mole) de N,N-diméthyl-1,2,3,5,6,7,8,8a-octahydro-5,5,8a-triméthyl-(6β,8aβ)-6-isoquinolineamine et de 2,6 g (1,9.$10^{-2}$ mole) de carbonate de potassium dans 40 ml d'acétonitrile.
A la fin de l'addition on agite le mélange réactionnel durant 2 heures puis on verse ce dernier dans de l'eau et on extrait avec l'ester éthylique de l'acide acétique. La phase organique est lavée avec de l'eau, séchée avec du sulfate de magnésium, filtrée et les solvants sont évaporés sous pression réduite.
Après purification sur une colonne de silice en éluant au moyen de 1,1'-oxybis-éthane on récupère 2,8 g (rendement : 52 %) du produit recherché sous forme d'huile jaune pâle.
$n^{23}_D$ = 1,5191

De façon analogue à la synthèse précédente, on prépare les composés suivants :

**Exemple 20**

**N,N-diméthyl-1,2,3,5,6,7,8,8a-octahydro-2-(2-propèn-1-yl)-5,5,8a-triméthyl-(6β,8aβ)-6-isoquinolineamine, (E)-2-butènedioate.**
F = 176°C

**Exemple 21**

**N,N-diméthyl-1,2,3,5,6,7,8,8a-octahydro-2-propyl-5,5,8a-triméthyl-(6β,8aβ)-6-isoquinolineamine, éthanedioate.**
F = 123-145°C

**Exemple 17**

**N,N-diméthyl-1,2,3,5,6,7,8,8a-octahydro-2-dodécyl-5,5,8a-triméthyl-(6β,8aβ)-6-isoquinolineamine, (E)-**

**2-butènedioate.**
F = 180°C

**Exemple 18**

**N,N-diméthyl-1,2,3,5,6,7,8,8a-octahydro-2-dodécyl-5,5,8a-triméthyl-(6α,8aβ)-6-isoquinolineamine, éthanedioate.**
F = 121-129°C

**Exemple 22**

**N,N-diméthyl-1,2,3,5,6,7,8,8a-octahydro-2-[1-(2,3-dihydroxypropyl)]-5,5,8a-triméthyl-(6β,8aβ)-6-isoquinolineamine.**
F = 74°C

**Exemple 23**

**N,N-diméthyl-1,2,3,5,6,7,8,8a-octahydro-2-(3-phényl-2-propèn-1-yl)-5,5,8a-triméthyl-(6β,8aβ)-6-isoquinolineamine, 4-méthylbensènesulfonate.**

A une solution de 0,4 g (1,6.$10^{-2}$ mole) d'acide 3-phényl-2-propénoïque dans 50 ml de tétrahydrofuranne, on ajoute 2,6 g (1,6.$10^{-2}$ mole) de 1,1'-carbonylbis-1H-imidazole et on agite une demi-heure à température ambiante puis environ 1 heure à 50°C. On refroidit la solution à 0°C et additionne 3 g (1,3.$10^{-2}$ mole) de N,N-diméthyl-1,2,3,5,6,7,8,8a-octahydro-5,5,8a-triméthyl-(6β,8aβ)-6-isoquinolineamine, en solution dans 40 ml de tétrahydrofuranne. On agite le mélange réactionnel pendant une nuit à température ambiante puis on le verse dans de l'eau et on extrait avec du 1,1'-oxybis-éthane. La phase organique est lavée à l'eau, séchée avec du sulfate de magnésium, filtrée et les solvants sont évaporés sous pression réduite. On purifie le produit récupéré par chromatographie sur colonne de silice en éluant au moyen du mélange dichlorométhane/méthanol/ammoniaque 98,5/1/0,5 (v/v/v). On recueille 2,8 g (rendement : 60 %) d'huile que l'on utilise brute dans l'étape suivante.

On solubilise 1,5 g (4,2.$10^{-3}$ mole) de l'huile précédente dans 30 ml de méthylbenzène puis on ajoute goutte à goutte 2,5 ml d'hydrure de bis (2-méthoxy-éthoxy) aluminium sodium à 70 % dans le méthylbenzène et on agite ce mélange pendant 2 heures et demie à température ambiante. On refroidit à environ 0°C et ajoute une solution d'hydroxyde de sodium 5N. Après agitation durant une demi-heure on extrait au 1,1'-oxybis-éthane. La phase organique est lavée à l'eau, séchée avec du sulfate de magnésium, filtrée et les solvants sont évaporés sous pression réduite.

On récupère une huile à partir de laquelle on prépare le 4-méthylbenzènesulfonate dans la 2-propanone. On obtient 1,5 g (rendement : 58 %) de produit après recristallisation dans l'acétonitrile.
F = 210°C

En procédant de manière analogue à la synthèse précédente, on prépare le produit suivant :

**Exemple 24**

**N,N-diméthyl-1,2,3,5,6,7,8,8a-octahydro-2-(3,3-diphényl-2-propèn-1-yl)-5,5,8a-triméthyl-(6β,8aβ)-6-isoquinolineamine, éthanedioate.**
F = 161°C

**Exemple 25**

**N,N-diméthyl-1,2,3,5,6,7,8,8a-octahydro-2-[1-[2-méthyl-3-[4-(1-méthyléthyl)phényl] propyl]] -5,5,8a-triméthyl-(6β,8aβ)-6-isoquinolineamine, (E)-2-butènedioate.**

A une solution de 3 g (1,3.$10^{-2}$ mole) de N,N-diméthyl-1,2,3,5,6,7,8,8a-octahydro-5,5,8a-triméthyl-(6β,8aβ)-6-isoquinolineamine dans 100 ml de méthanol on ajoute 2,8 g (1,5.$10^{-2}$ mole) de 2-méthyl-3-[4-(1-méthyléthyl)phényl]propanal et 2 g de sulfate de sodium. Après addition d'acide acétique pour ajuster le pH du mélange à environ 7, on ajoute 1,3 g (2.$10^{-2}$ mole) de cyanotriborohydrure de sodium et on agite ce mélange une nuit à température ambiante. Après filtration et évaporation des solvants on reprend le résidu par une solution normale d'hydroxyde de sodium. On extrait au 1,1'-oxybis-éthane. La phase organique est lavée à l'eau, séchée, filtrée et les solvants sont évaporés sous pression réduite. Le produit obtenu est purifié par chromatographie sur une colonne de silice en éluant au moyen du mélange dichlorométhane/méthanol/ ammoniaque 95,5/4/0,5 (v/v/v). On recueille 2,2 g d'huile (rendement : 41%) à partir de laquelle on prépare le (E)-2-butènedioate recherché dans la 2-propanone.
F = 142-150°C

On a regroupé dans le tableau I suivant un certain nombre de composés selon l'invention. Dans ce tableau les symboles utilisés ont les significations suivantes :

n-$C_{12}$ : $-(CH_2)_{11}-CH_3$

A :

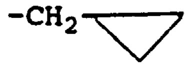

B :

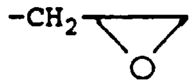

C :

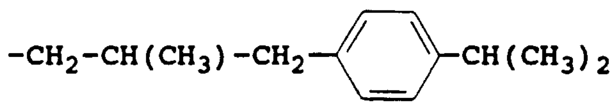

D :

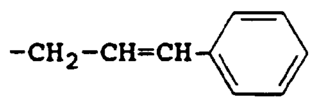

E :

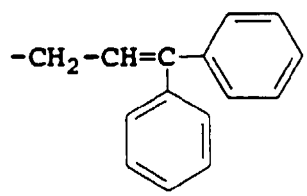

F : $-CH_2-CH=CH-C\equiv C-C(CH_3)_3$

Les symboles utilisés pour les sels ont les significations suivantes :

Oxal : $HO_2C-CO_2H$

Fum : $HO_2C-CH=CH-CO_2H$ (E)

$TS0_3H$ :

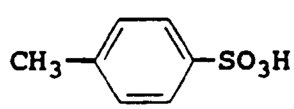

Base : composé sous forme de base.

TABLEAU I

| Ex | $R_1$ | $R_2$ | $R_3$ | Sel |
|---|---|---|---|---|
| 1 | $-CH_2-CH=CH_2$ | H | $CF_3CO$ | Base |
| 2 | $-CH_2-CH_2-CH_3$ | H | $CF_3CO$ | Base |
| 3 | A | H | $CF_3CO$ | Fum |

TABLEAU I (suite)

| Ex | $R_1$ | $R_2$ | $R_3$ | Sel |
|---|---|---|---|---|
| 4 | F | H | $CF_3CO$ | Base |
| 5 | B | H | $CF_3CO$ | Base |
| 6 | $-CH_2-CH(OH)-CH_2-OH$ | H | $CF_3CO$ | Fum |
| 7 | $-CH_2-CH=C(CH_3)_2$ | H | $CF_3CO$ | Oxal |
| 8 | $n-C_{12}$ | H | $CF_3CO$ | Base |
| 9* | $-CH_2-CH=CH_2$ | H | $CF_3CO$ | Base |
| 10 | $n-C_{12}$ | $CH_3$ | $CF_3CO$ | Base |
| 11 | $-CH_2-CH=CH_2$ | $CH_3$ | $CF_3CO$ | Base |
| 12 | $n-C_{12}$ | $CH_3$ | H | Oxal |
| 13 | $-CH_2-CH=CH_2$ | $CH_3$ | H | Oxal |
| 14 | $n-C_{12}$ | H | H | Fum |
| 15 | $-CH_2-CH=CH_2$ | H | H | $TSO_3H$ |
| 16 | $-CH_2-CH=CH_2$ | H | $CF_3CO$ | $TSO_3H$ |
| 17 | $n-C_{12}$ | $CH_3$ | $CH_3$ | Fum |
| 18* | $n-C_{12}$ | $CH_3$ | $CH_3$ | Oxal |
| 19 | F | $CH_3$ | $CH_3$ | Base |
| 20 | $-CH_2-CH=CH_2$ | $CH_3$ | $CH_3$ | Fum |
| 21 | $-CH_2-CH_2-CH_3$ | $CH_3$ | $CH_3$ | Oxal |
| 22 | $-CH_2-CH(OH)-CH_2-OH$ | $CH_3$ | $CH_3$ | Base |
| 23 | D | $CH_3$ | $CH_3$ | $TS0_3H$ |
| 24 | E | $CH_3$ | $CH_3$ | Oxal |
| 25 | C | $CH_3$ | $CH_3$ | Fum |

*Composés avec une liaison 6α, tous les autres ont une liaison 6β.

Les produits selon l'invention sont des inhibiteurs de la biosynthèse du cholestérol et notamment de l'époxysqualène cyclase.

L'activité des composés selon l'invention a été évaluée par mise en évidence d'un effet inhibiteur sur l'époxysqualène cyclase des microsomes hépatiques de rats mâles Wistar.

La méthode consiste à mesurer le lanostérol formé à partir du R,S-2,3-oxydosqualène par l'enzyme microsomiale. La préparation de l'enzyme se fait selon la méthode décrite par Ness G.C (Ness G.C. et al. Biochem. J (1986) 233,167-172).

Méthode de mesure de l'activité époxysqualène cyclase :

Les microsomes hépatiques de rat sont utilisés comme source d'enzyme. La méthode consiste à mesurer le lanostérol formé à partir du R,S-2,3-oxydosqualène. Le R,S-2,3-oxydosqualène, les produits à tester et le Tween$^R$80 sous forme de solutions organiques (2-propanone ou sulfinylbis-méthane) (25 µl) sont mis dans les tubes d'essai puis 400 µl de tampon phosphate de potassium (0,1 M, pH = 7,4) sont ajoutés. La réaction est initiée par addition de 100 µl de microsomes. Pour un volume réactionnel final de 525 µl, le mélange contient 150 µM de R,S-2,3-oxydosqualène, 0,1 % de Tween$^R$80 (pour solubiliser le R,S-2,3-oxydosqualène) et 250 µg de protéines microsomiales. La durée de réaction est de 60 minutes à 37°C. La réaction est stoppée par addition de 300 µl de potasse méthanolique (7 %) et de 20 µg de stigmastérol comme standard interne. Après saponification à 80°C pendant 30 minutes et agitation à l'agitateur rotatif, les stérols sont extraits au moyen de 2 ml d'hexane.

Le lanostérol formé est séparé du R,S-2,3-oxydosqualène, du cholestérol membranaire et du stigmastérol par chromatographie en phase gazeuse après transformation en éthers triméthylsilylés. La dérivatisation des stérols s'effectue à 60°C pendant 30 minutes après addition de 25 µl de pyridine et 75 µl de l'ester triméthylsilique de l'acide 2,2,2-trifluoro-N-triméthylsilyl-éthanimidique contenant 1 % d'éthers triméthylchlorosilanes.
Après évaporation, les éthers triméthylsilylés sont remis en solution dans 100 µl d'hexane. Une partie aliquote de cette solution (2 µl) est chromatographiée en phase gazeuse sur colonne capillaire OV1 (0,32 mm, 25 m) dans les conditions suivantes : température de l'injecteur = 270°C, température du four = 260°C, température du détecteur = 300°C, le gaz vecteur étant l'azote à une pression de $7.10^4$ Pa.

La puissance des molécules testées est exprimée en pourcentage d'inhibition de la quantité de lanostérol formé pour une concentration de $25.10^{-6}$ mole par litre du produit testé. Les résultats obtenus avec un certain nombre de composés selon l'invention sont regroupés dans le tableau II.

TABLEAU II

| Ex | Pourcentage d'inhibition pour $25.10^{-6}$ mole de produit testé |
|---|---|
| 6 | 62 |
| 12 | 65 |
| 13 | 63 |
| 14 | 29 |
| 17 | 100 |
| 18 | 66 |
| 20 | 89 |
| 21 | 100 |
| 22 | 64 |
| 23 | 98 |
| 24 | 92 |
| 25 | 98 |

L'activité des composés selon l'invention a été aussi évaluée par la mise en évidence de leur capacité à inhiber la biosynthèse du cholestérol in vivo et notamment à inhiber l'époxysqualène cyclase hépatique chez la souris OF1 suivant le protocole opératoire décrit ci-après :

L'influence des molécules sur la cholestérogénèse hépatique in vivo est testée chez la souris OF1 mâle. Les animaux sont conditionnés en cycle inversé deux semaines avant l'administration du produit.

Le jour de l'expérience, les animaux (3 à 6 souris/groupe) reçoivent à T=0 une administration par voie orale (gavage) du produit testé en suspension dans le véhicule (gomme arabique à 3 % dans l'eau).
A T=1h, le précurseur radioactif (RS) 2-$^{14}$C mévalonolactone) en solution aqueuse à 0,9 % de NaCl est injecté par voie intra-péritonéale [2,5 ou 5 µCi/souris (soit 9,25 x $10^4$ Bq ou respectivement 1,85 x $10^5$ Bq)].
A T=2h, les animaux sont euthanasiés par dislocation cervicale ; les foies sont prélevés, rincés, pesés et congelés dans l'azote liquide avant stockage à -20°C.

Durant la période de conditionnement ainsi que le jour de l'expérience, les animaux ont libre accès à la nourriture et à l'eau de boisson.
L'administration du produit testé et l'injection du précurseur radioactif ont lieu en milieu de la période d'obscurité.

Après décongélation, le foie total (ou une partie du foie) est homogénéisé dans l'eau à 0-4°C. Une partie aliquote de l'homogénat est alors saponifiée dans la potasse alcoolique durant 90 minutes à 80°C. Les lipides non saponifiables sont ensuite extraits par l'éther de pétrole. Le solvant est évaporé à sec sous flux d'azote et le résidu sec est redissous dans un mélange chloroforme/méthanol (2/1). Les produits contenus dans l'extrait sont alors séparés par chromatographie sur couche mince de silice après migration dans le système hexane/ester éthylique de l'acide acétique (80/20).

Le dépôt d'un mélange standard contenant : cholestérol, lanostérol, mono-époxysqualène, di-époxysqualène et squalène froids plus du $^{14}$C-cholestérol, permet de déterminer le Rf des composés qui servent de référence pour l'analyse du profil radioactif des lipides non saponifiables.

La répartition de la radioactivité est analysée par un Analyseur Linéaire Automatique. Le pourcentage relatif de la radioactivité du pic des $C_{27}$-stérols du groupe témoin (véhicule) représente le 100 % d'incorporation. Le calcul d'influence est basé sur le rapport du pourcentage de radioactivité du pic de $C_{27}$-stérols du groupe traité sur celui du groupe témoin.

Cette méthode a permis de mettre en évidence une inhibition de la biosynthèse du cholestérol in vivo avec certaines

molécules par la mesure de la baisse d'incorporation de radioactivité dans le pic des $C_{27}$-stérols associée à une augmentation de radioactivité dans les pics correspondant aux époxysqualènes , ainsi que la présence de mono-époxysqualène identifié par chromatographie gazeuse couplée avec un spectromètre de masse (GC/MS).

Les résultats obtenus avec un certain nombre de composés selon l'invention sont regroupés dans le tableau III et montrent que certaines molécules de cette série chimique sont des inhibiteurs potentiels de l'époxysqualène cyclase hépatique de la souris OF1 in vivo.

Les produits selon l'invention sont utiles en thérapeutique dans le traitement et la prévention de l'hypercholestérolémie, notamment des phénomènes de lésions artérielles qui y sont associés tel l'athérosclérose, et des mycoses et autres affections parasitaires provoquées par un champignon comme par exemple Actinomyces mentagrophytes, Candida tropicalis, Candida albicans, Candida glabrata ou Aspergillus fumigatus.

Selon l'invention on préconise une composition thérapeutique caractérisée en ce qu'elle renferme au moins un composé de formule I ou l'un de ses sels d'addition d'acide non-toxiques en quantité thérapeutiquement efficace, en association avec un excipient physiologiquement acceptable.

On préconise également l'utilisation des composés de formule I ou l'un de leurs sels d'addition d'acide non-toxiques, en tant qu'agents inhibiteurs de l'époxysqualène cyclase, pour l'obtention d'un médicament préventif ou curatif hypocholestérolémiant, hypolipémiant, antiathéromateux et/ou antifongique.

Les produits de formule I selon l'invention et leurs sels d'addition sont en particulier utiles dans le traitement des D.I.C. (coagulations intravasculaires disséminées) induites notamment par les moisissures telles que Candida albicans et Candida glabrata.

Le meilleur mode de mise en oeuvre de l'invention consiste à utiliser les produits des exemples 1, 17, 23 et 25 en tant que médicaments notamment hypocholestérolémiants et/ou antifongiques.

TABLEAU III

| Influence des composés de formule I ou de leurs sels sur la biosynthèse du cholestérol hépatique chez la souris OF1 mâle. | | |
|---|---|---|
| Ex | Dose en mg base/kg | Influence en pourcentage* |
| 1 | 50 | - 37 |
| 1 | 100 | - 82/- 70 |
| 5 | 50 | - 32 |
| 6 | 100 | - 74/- 78 |
| 16 | 50 | - 14 |
| 17 | 50 | - 81/- 66 |
| 20 | 50 | - 21 |

* influence sur l'incorporation de radioactivité dans les $C_{27}$-stérols.

**Revendications**

1. Composé 1,2,3,5,6,7,8,8a-octahydro-5,5,8a-triméthyl-(8aβ)-6-isoquinolineamine, caractérisé en ce qu'il est choisi parmi l'ensemble constitué par :

(i) les composés de formule :

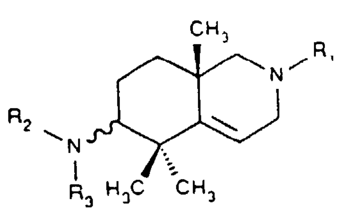

(I)

dans laquelle :

- $R_1$ représente :

  - un groupe alkyle, linéaire ou ramifié, en $C_1$-$C_{12}$ éventuellement substitué par

    * un groupe cycloalkyle en $C_3$-$C_6$,
    * un groupe oxirannyle,
    * un groupe phényle substitué par un groupe alkyle en $C_1$-$C_4$,

  - un groupe alkyle, linéaire ou ramifié, en $C_3$-$C_{12}$ comportant une ou plusieurs liaisons doubles ou triples, éventuellement substitué par un ou deux groupes phényle,

  - un groupe alkyle en $C_3$-$C_4$, linéaire ou ramifié, substitué par un ou plusieurs groupes hydroxyle,

- soit $R_2$ et $R_3$, identiques ou différents, représentent chacun un atome d'hydrogène ou un groupe alkyle en $C_1$-$C_4$, soit $R_2$ représente un atome d'hydrogène ou un groupe alkyle en $C_1$-$C_4$ et $R_3$ représente un groupe trifluoroacétyle ou un groupe acétyle,
- le symbole ∿∿ représente une liaison α ou une liaison β en position 6, et

(ii) leurs sels d'addition d'acide.

**2.** Composé selon la revendication 1, caractérisé en ce que le symbole ∿∿ représente une liaison α en position 6.

**3.** Composé selon la revendication 1, caractérisé en ce que le symbole ∿∿ représente une liaison β en position 6.

**4.** Composé selon la revendication 1, caractérisé en ce qu'il répond à la nomenclature de N-trifluoroacétyl-1,2,3,5,6,7,8,8a-octahydro-2-(2-propèn-1-yl)-5,5,8a-triméthyl-(6β,8aβ)-6-isoquinolineamine.

**5.** Composé selon la revendication 1, caractérisé en ce qu'il répond à la nomenclature de N,N-diméthyl-1,2,3,5,6,7,8,8a-octahydro-2-dodécyl-5,5,8a-triméthyl-(6β,8aβ)-6-isoquinolineamine, (E)-2-butènedioate.

**6.** Composé selon la revendication 1, caractérisé en ce qu'il répond à la nomenclature de N,N-diméthyl-1,2,3,4,5,6,7,8,8a-octahydro-2-(3-phényl-2-propèn-1-yl)-5,5,8a-triméthyl-(6β,8aβ)-6-isoquinolineamine, 4-méthyl-benzènesulfonate.

**7.** Composé selon la revendication 1, caractérisé en ce qu'il répond à la nomenclature de N,N-diméthyl-1,2,3,5,6,7,8,8a-octahydro-2-[1-[2-méthyl-3-[4-(1-méthyl-éthyl)phényl]propyl]]-5,5,8a-triméthyl-(6β,8aβ)-6-isoquinolineamine, (E)-2-butènedioate.

**8.** Procédé de préparation d'un composé de formule I selon la revendication 1 ou de l'un de ses sels d'addition d'acide, ledit procédé étant caractérisé en ce qu'il comprend les étapes selon lesquelles :

i) on soumet à une N-alkylation un composé de formule :

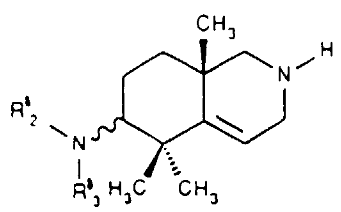

(II)

dans laquelle :
le symbole ∿∿ représente une liaison α ou une liaison β en position 6 et, soit $R'_2$ et $R'_3$, identiques ou différents, représentent chacun un groupe alkyle en $C_1$-$C_4$, soit $R'_2$ représente un atome d'hydrogène et $R'_3$ représente un groupe trifluoroacétyle, par réaction avec un composé de formule $R'_1$-X dans laquelle X représente un atome d'halogène et $R'_1$ a la même définition que $R_1$ dans la formule I, à raison de 1 mole de composé de formule II pour 1,1 à 1,2 moles de composé de formule $R'_1$-X à une température comprise entre la température ambiante et 150°C et pendant au moins une heure pour obtenir un composé de formule :

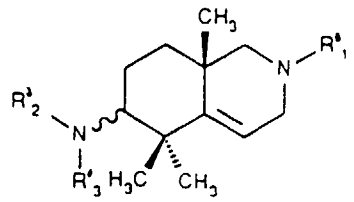

(I')

dans laquelle $R'_1$, $R'_2$ et $R'_3$ sont définis comme ci-dessus et le symbole ∿∿ représente une liaison α ou une liaison β en position 6 ;

ii) si nécessaire, on soumet les composés de formule I' ainsi obtenus à au moins un des traitements suivants :

a) les composés de formule I' dans laquelle $R'_1$ est défini comme précédemment, le symbole ∿∿ représente une liaison α ou une liaison β en position 6, $R'_2$ représente un atome d'hydrogène et $R'_3$ représente un groupe trifluoroacétyle sont transformés, par alkylation de l'amide en position 6 par réaction avec un halogènure d'alkyle en $C_1$-$C_4$, en présence d'une base forte, à raison de une mole de composé de formule I' pour 1,2 moles d'halogènure d'alkyle à la température ambiante, en composés de formule I dans laquelle $R_1$ est défini comme précédemment, le symbole ∿∿ représente une liaison α ou une liaison β en position 6, $R_2$ représente un groupe alkyle en $C_1$-$C_4$ et $R_3$ représente un groupe trifluoroacétyle ;

b) on élimine le groupe trifluoroacétyle porté par l'amine en position 6 des composés de formule I' dans laquelle $R'_1$ a la même définition que précédemment, le symbole ∿∿ représente une liaison α ou une liaison β en position 6 et $R'_3$ représente un groupe trifluoroacétyle et $R'_2$ représente un atome d'hydrogène ou un groupe alkyle en $C_1$-$C_4$, par hydrolyse par un sel de métal alcalin dans un alcool pour obtenir un composé de formule I dans laquelle $R_1$ a la même définition que précédemment, le symbole ∿∿ représente une liaison α ou une liaison β en position 6, $R_3$ représente un atome d'hydrogène et $R_2$ représente un atome d'hydrogène ou un groupe alkyle en $C_1$-$C_4$ ;

c) on acyle les composés obtenus en b) par réaction avec un anhydride d'acide pour obtenir les composés de formule I dans laquelle $R_1$ a la même définition que précédemment, à l'exception des composés de formule I où $R_1$ représente un groupe alkyle en $C_3$-$C_4$, linéaire ou ramifié, substitué par un ou plusieurs groupes hydroxyles ou encore un groupe alkyle, linéaire ou ramifié, en $C_1$-$C_{12}$ substitué par un groupe oxirannyle, $R_3$ représente un groupe acyle, $R_2$ représente un atome d'hydrogène ou un groupe alkyle en $C_1$-$C_4$ et le symbole ∿∿ représente une liaison α ou une liaison β en position 6 ;

d) les composés obtenus en b) dans lesquels $R_2$ et $R_3$ représentent tous deux un atome d'hydrogène sont transformés par alkylation réductrice au moyen d'un dérivé carbonylé approprié puis réduction de l'ion iminium formé par un agent réducteur, en composés de formule I dans laquelle $R_2$ et $R_3$, identiques ou différents, représentent chacun un groupe alkyle en $C_1$-$C_4$, $R_1$ a la même définition que précédemment et le symbole ∿∿ représente soit une liaison α soit un liaison β en position 6.

**9.** Composé intermédiaire, utile dans la synthèse des composés de formule I selon la revendication 1, caractérisé en ce qu'il s'agit d'un produit de formule :

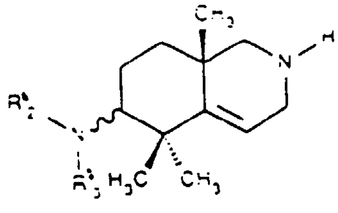

(II)

dans laquelle le symbole ∿∿ représente une liaison α ou une liaison β en position 6 et, soit $R'_2$ représente un

atome d'hydrogène et $R'_3$ représente un groupe trifluoroacétyle, soit $R'_2$ et $R'_3$, identiques ou différents, représentent chacun un groupe alkyle en $C_1$-$C_4$.

**10.** Composé intermédiaire, utile dans la synthèse de composé de formule II selon la revendication 9, caractérisé en ce qu'il s'agit d'un produit de formule :

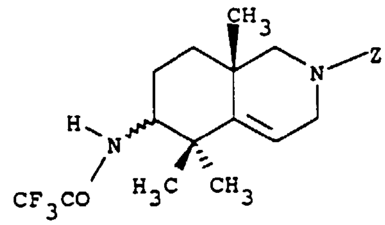

(VI)

dans laquelle le symbole ∿∿ représente une liaison α ou une liaison β en position 6 et Z représente un groupe protecteur.

**11.** Composition thérapeutique caractérisée en ce qu'elle renferme au moins un composé de formule I ou l'un de ses sels d'addition d'acide non-toxiques selon la revendication 1, en quantité thérapeutiquement efficace, en association avec un excipient physiologiquement acceptable.

**12.** Utilisation d'un composé de formule I ou de l'un de ses sels d'addition d'acide non-toxiques selon la revendication 1, en tant qu'agent inhibiteur de la biosynthèse du cholestérol pour l'obtention d'un médicament préventif ou curatif de l'hypercholestérolémie, l'athérosclérose et des mycoses.

**Patentansprüche**

**1.** 1,2,3,5,6,7,8,8a-Octahydro-5,5,8a-trimethyl(8aβ)-6-isocuinolinamin, dadurch gekennzeichnet, daß es ausgewählt ist aus der Gruppe bestehend aus:

(i) den Verbindungen der Formel:

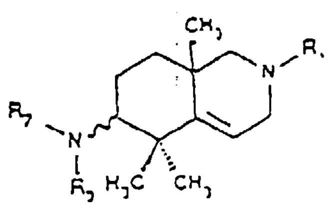

(I)

worin $R_1$ bedeutet:

- eine lineare oder verzweigte $C_1$-$C_{12}$-Alkylgruppe, die gegebenenfalls substituiert ist durch

  eine $C_3$-$C_6$-Cycloalkylgruppe,
  eine Oxiranylgruppe,
  eine durch eine $C_1$-$C_4$-Alkylgruppe substituierte Phenylgruppe,

- eine lineare oder verzweigte $C_3$-$C_{12}$-Alkylgruppe, die eine oder mehrere Doppel- oder Dreifachbindungen aufweist, und gegebenenfalls durch ein oder zwei Phenylgruppen substituiert ist;
- eine geradkettige oder verzweigte $C_3$-$C_4$-Alkylgruppe, die durch eine oder mehrere Hydroxylgruppen substituiert ist;
- und $R_2$ und $R_3$, die gleich oder verschieden sein können, jeweils ein Wasserstoffatom oder eine $C_1$-$C_4$-Alkylgruppe darstellen, oder $R_2$ ein Wasserstoffatom oder eine $C_1$-$C_4$-Alkylgruppe bedeutet und $R_3$ eine Trifluoracetylgruppe oder eine Acetylgruppe bedeutet, wobei das Symbol ∿∿ eine α- oder eine β-Bindung in Stellung 6 bedeutet, und (ii) ihren Säureadditionssalzen.

**2.** Verbindung nach Anspruch 1, dadurch gekennzeichnet, daß das Symbol eine $\alpha$-Bindung in 6-Stellung bedeutet.

**3.** Verbindung nach Anspruch 1, dadurch gekennzeichnet, daß das Symbol eine $\beta$-Bindung in 6-Stellung bedeutet.

**4.** Verbindung nach Anspruch 1, dadurch gekennzeichnet, daß sie N-Trifluoracetyl-1,2,3,5,6,7,8,8a-octahydro-2-(2-propen-1-yl)-5,5,8a-trimethyl-(6β,8aβ)-6-isocuinolinamin ist.

**5.** Verbindung nach Anspruch 1, dadurch gekennzeichnet, daß sie N,N-Dimethyl-1,2,3,5,6,7,8,8a-Octahydro-2-dodecyl-5,5,8a-trimethyl-(6β,8aβ)-6-isocuinolinamin-(E)-2-Butendioat ist.

**6.** Verbindung nach Anspruch 1, dadurch gekennzeichnet, daß sie N,N-Dimethyl-1,2,3,4,5,6,7,8,8a-Octahydro-2-(3-phenyl-2-propen-1-yl)-5,5,8a-trimethyl-(6β,8aβ)-6-isocuinolinamin-4-Methylbenzolsulfonat ist.

**7.** Verbindung nach Anspruch 1, dadurch gekennzeichnet, daß sie N,N-Dimethyl-1,2,3,5,6,7,8,8a-Octahydro-2-[1-[2-methyl-3-[4-(1-methyl-ethyl)phenyl]propyl]]-5,5,8a-trimethyl-(6β,8aβ)-6-isocuinolinamin-(E)-2-Butendioat ist.

**8.** Verfahren zur Herstellung einer Verbindung der Formel I gemäß Anspruch 1 oder eines Säureadditionssalzes davon, dadurch gekennzeichnet, daß es die Stufen umfaßt, in denen man:

(i) eine Verbindung der Formel

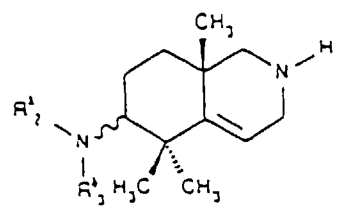

(II)

worin:
das Symbol ∿ eine $\alpha$- oder $\beta$-Bindung in 6-Stellung bedeutet, und $R'_2$ und $R'_3$, die gleich oder verschieden sein können, jeweils eine $C_1$-$C_4$-Alkylgruppe bedeuten, oder $R'_2$ ein Wasserstoffatom und $R'_3$ eine Trifluoracetylgruppe bedeutet, einer N-Alkylierung durch Umsetzung mit einer Verbindung der Formel $R'_1$-X, worin X ein Halogenatom bedeutet und $R'_1$ die gleiche Bedeutung wie $R_1$ in Formel I besitzt, im Verhältnis von 1 Mol der Verbindung der Formel II pro 1,1 bis 1,2 Mol der Verbindung der Formel $R'_1$-X bei einer Temperatur zwischen Raumtemperatur und 150 °C während einer Stunde unterwirft, um eine Verbindung der Formel

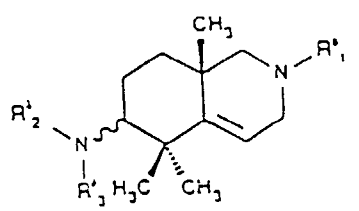

(I')

zu erhalten, worin $R'_1$, $R'_2$ und $R'_3$ die vorstehend angegebene Bedeutung besitzen und das Symbol ∿ eine $\alpha$- oder $\beta$-Bindung in 6-Stellung bedeutet;
(ii) wenn notwendig, die so erhaltenen Verbindungen der Formel I' mindestens einer der folgenden Behandlungen unterwirft:

(a) die Verbindungen der Formel I', worin $R'_1$ die vorstehend angegebene Bedeutung besitzt, das Symbol ∿ eine $\alpha$- oder $\beta$-Bindung in 6-Stellung bedeutet, $R'_2$ ein Wasserstoffatom bedeutet und $R'_3$ eine Trifluoracetylgruppe bedeutet, werden durch Aminoalkylierung in 6-Stellung durch Umsetzung mit einem $C_1$-$C_4$-Alkylhalogenid in Gegenwart einer starken Base in einem Verhältnis von 1 Mol der Verbindung der Formel I' pro 1,2 Mole Alkylhalogenid bei Raumtemperatur, in die Verbindungen der Formel I, worin $R_1$

die vorstehend angegebene Bedeutung besitzt, das Symbol eine ∿∿ α- oder β-Bindung in 6-Stellung bedeutet, $R_2$ eine $C_1$-$C_4$-Alkylgruppe bedeutet, und $R_3$ eine Trifluoracetylgruppe bedeutet, überführt;

(b) man eliminiert die Trifluoracetylgruppe des Amins in 6-Stellung der Verbindungen der Formel I', worin $R'_1$ die vorstehend angegebene Bedeutung besitzt, das Symbol ∿∿ eine α- oder eine β-Bindung in 6-Stellung bedeutet, und $R'_3$ eine Trifluoracetylgruppe bedeutet und $R'_2$ ein Wasserstoffatom oder eine $C_1$-$C_4$-Alkylgruppe bedeutet, durch Hydrolyse mittels eines Alkalimetallsalzes in einem Alkohol, um eine Verbindung der Formel I zu erhalten, worin $R_1$ die vorstehend angegebene Bedeutung besitzt, das Symbol ∿∿ eine α- oder β-Bindung in 6-Stellung bedeutet, $R_3$ ein Wasserstoffatom bedeutet und $R_2$ ein Wasserstoffatom oder eine $C_1$-$C_4$-Alkylgruppe bedeutet;

(c) man acyliert die unter (b) erhaltenen Verbindungen durch Umsetzung mit einem Säureanhydrid, um die Verbindungen der Formel I zu erhalten, worin $R_1$ die vorstehend angegebene Bedeutung besitzt, mit Ausnahme der Verbindungen der Formel I, worin $R_1$ eine lineare oder verzweigte, durch ein oder mehrere Hydroxylgruppen substituierte $C_3$-$C_4$-Alkylgruppe bedeutet, oder eine lineare oder verzweigte durch eine Oxiranylgruppe substituierte $C_1$-$C_{12}$-Alkylgruppe bedeutet, $R_3$ eine Acylgruppe bedeutet, $R_2$ ein Wasserstoffatom oder eine $C_1$-$C_4$-Alkylgruppe bedeutet, und das Symbol eine α- oder eine β-Bindung in 6-Stellung bedeutet;

(d) man überführt die unter (b) erhaltenen Verbindungen, in denen $R_2$ und $R_3$ beide ein Wasserstoffatom bedeuten, durch reduktive Alkylierung mittels eines geeigneten Carbonylderivates und nachfolgender Reduktion des gebildeten Iminiumions mittels eines Reduktionsmittels in Verbindungen der Formel I, worin $R_2$ und $R_3$, die gleich oder verschieden sein können, jeweils eine $C_1$-$C_4$-Alkylgruppe bedeuten, $R_1$ die vorstehend angegebene Bedeutung besitzt, und das Symbol ∿∿ eine α- oder β-Bindung in 6-Stellung bedeutet.

**9.** Zwischenprodukt zur Verwendung zur Synthese der Verbindungen der Formel I gemäß Anspruch 1, dadurch gekennzeichnet, daß es ein Produkt der Formel ist:

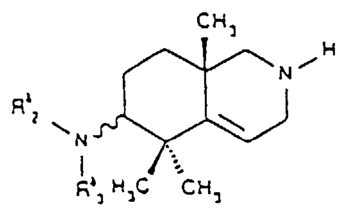

(II)

worin das Symbol ∿∿ eine α- oder β-Bindung in 6-Stellung bedeutet, $R'_2$ ein Wasserstoffatom und $R'_3$ eine Trifluoracetylgruppe bedeutet, oder $R'_2$ und $R'_3$, die gleich oder verschieden sein können, jeweils eine $C_1$-$C_4$-Alkylgruppe bedeuten.

**10.** Zwischenprodukt zur Verwendung zur Synthese der Verbindungen der Formel II gemäß Anspruch 9, dadurch gekennzeichnet, daß es ein Produkt der Formel ist:

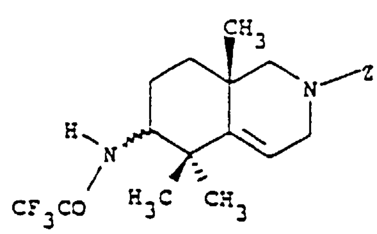

(VI)

worin das Symbol ∿∿ eine α- oder β-Bindung in 6-Stellung bedeutet, und Z eine Schutzgruppe bedeutet.

**11.** Therapeutische Zusammensetzung, dadurch gekennzeichnet, daß sie mindestens eine Verbindung der Formel I oder eines ihrer nicht toxischen Säureadditionssalze gemäß Anspruch 1 in einer therapeutisch wirksamen Menge zusammen mit einem physiologisch annehmbaren Träger enthält.

**12.** Verwendung einer Verbindung der Formel I oder eines seiner nicht-toxischen Säureadditionssalze gemäß Anspruch 1 als Inhibitor der Biosynthese von Cholesterin, um ein Arzneimittel zur Vorbeugung oder Heilung von Hypercholesterinämie, Atherosklerose und Mycosen zu erhalten.

## Claims

**1.** A 1, 2, 3, 5, 6, 7, 8, 8a-octahydro-5, 5, 8a-trimethyl-(8aβ)-6-isoquinolineamine compound, characterized in that it is selected from the group consisting of:

(i) the compounds of the formula

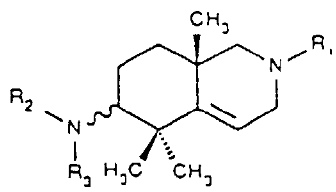

(I)

in which

- $R_1$ is :
  - a linear or branched $C_1$-$C_{12}$-alkyl group optionally substituted by :
    - * a $C_3$-$C_6$-cycloalkyl group,
    - * an oxiranyl group, or
    - * a phenyl group substituted by a $C_1$-$C_4$-alkyl group,
  - a linear or branched $C_3$-$C_{12}$-alkyl group containing one or more double or triple bonds and optionally substituted by one or two phenyl groups, or
  - a linear or branched $C_3$-$C_4$-alkyl group substituted by one or more hydroxyl groups,
- either $R_2$ and $R_3$, which are identical or different, are each a hydrogen atom or a $C_1$-$C_4$-alkyl group, or $R_2$ is a hydrogen atom or a $C_1$-$C_4$-alkyl group and $R_3$ is a trifluoroacetyl group or an acetyl group, and
- the symbol ∿∿ is an α-bond or a β-bond in the 6-position, and

(ii) their acid addition salts.

**2.** A compound according to claim 1, characterized in that the symbol ∿∿ is an α-bond in the 6-position.

**3.** A compound according to claim 1, characterized in that the symbol ∿∿ is a β-bond in the 6-position.

**4.** A compound according to claim 1, characterized in that it corresponds to the nomenclature N-trifluoro-acetyl-1, 2, 3, 5, 6, 7, 8, 8a-octahydro-2-(2-propen-1-yl)-5, 5, 8a-trimethyl-(6β, 8aβ)-6-isoquinoline-amine.

**5.** A compound according to claim 1, characterized in that it corresponds to the nomenclature N, N-dimethyl-1, 2, 3, 5, 6, 7, 8, 8a-octahydro-2-dodecyl-5, 5, 8a-trimethyl-(6β, 8aβ)-6-isoquinolineamine (E)-2-butenedioate.

**6.** A compound according to claim 1, characterized in that it corresponds to the nomenclature N, N-dimethyl-1, 2, 3, 5, 6, 7, 8, 8a-octahydro-2-(3-phenyl-2-propen-1-yl)-5, 5, 8a-trimethyl-(6β, 8aβ)-6-isoquinolineamine 4-methylbenzenesulfonate.

**7.** A compound according to claim 1, characterized in that it corresponds to the nomenclature N, N-dimethyl-1, 2, 3, 5, 6, 7, 8, 8a-octahydro-2-[1-[2-methyl-3-[4-(1-methylethyl)phenyl]propyl]]-5, 5, 8a-trimethyl-(6β, 8aβ)-6-isoquinolineamine (E)-2-butenedioate.

**8.** A method of preparing a compound of formula I according to claim 1, or one of its acid addtion salts, said method being characterized in that it comprises the steps wherein :

i) a compound of the formula

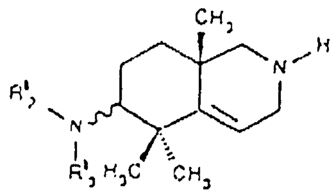

(II)

in which
the symbol ∿ is an α-bond or a β-bond in the 6-position and either $R'_2$ and $R'_3$, which are identical or different, are each a $C_1$-$C_4$-alkyl group, or $R'_2$ is a hydrogen atom and $R'_3$ is a trifluoroacetyl group, is N-alkylated by reaction with a compound of the formula $R'_1$-X, in which X is a halogen atom and $R'_1$ is defined in the same way as $R_1$ in formula I, at a rate of 1 mol of compound of formula II to 1.1 to 1.2 mol of compound of the formula $R'_1$-X, at a temperature between room temperature and 150°C and for at least one hour, to give a compound of the formula

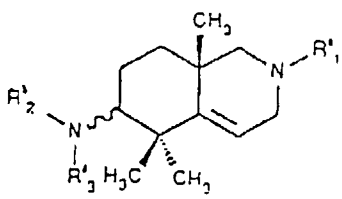

(I')

in which $R'_1$, $R'_2$ and $R'_3$ are as defined above and the symbol ∿ is an α-bond or a β-bond in the 6-position ; and
ii) if necessary, the resulting compounds of formula I' are subjected to at least one of the following treatments :

a) the compounds of formula I' in which $R'_1$ is as defined above, the symbol ∿ is an α-bond or a β-bond in the 6-position, $R'_2$ is a hydrogen atom and $R'_3$ is a trifluoroacetyl group are converted, by alkylation of the amide in the 6-position by reaction with a $C_1$-$C_4$-alkyl halide, in the presence of a strong base, at a rate of one mol of compound of formula I' to 1.2 mol of alkyl halide, at room temperature, into compounds of formula I in which $R_1$ is as defined above, the symbol ∿ is an α-bond or a β-bond in the 6-position, $R_2$ is a $C_1$-$C_4$-alkyl group and $R_3$ is a trifluoroacetyl group ;
b) the trifluoroacetyl group carried by the amine in the 6-position of the compounds of formula I' in which $R'_1$ is as defined above, the symbol ∿ is an α-bond or a β-bond in the 6-position, $R'_3$ is a trifluoroacetyl group and $R'_2$ is hydrogen atom or a $C_1$-$C_4$-alkyl group is removed by hydrolysis with an alkali metal salt in an alcohol to give a compound of formula I in which $R_1$ is as defined above, the symbol ∿ is an α-bond or a β-bond in the 6-position, $R'_3$ is a hydrogen atom and $R_2$ is a hydrogen atom or a $C_1$-$C_4$-alkyl group ;
c) the compounds obtained in b) are acylated by reaction with an acid anhydride to give the compounds of formula I in which $R_1$ is as defined above, with the exception of the compounds of formula I in which $R_1$ is a linear or branched $C_3$-$C_4$-alkyl group substituted by one or more hydroxy groups, or else a linear or branched $C_1$-$C_{12}$-alkyl group substituted by an oxiranyl group, $R_3$ is an acyl group, $R_2$ is a hydrogen atom or a $C_1$-$C_4$-alkyl group and the symbol ∿ is an α-bond or a β-bond in the 6-position ; or
d) the compounds obtained in b) in which $R_2$ and $R_3$ are both a hydrogen atom are converted, by reductive alkylation with an appropriate carbonyl derivative, followed by reduction of the iminium ion formed with a reducing agent, into compounds of formula I in which $R_2$ and $R_3$, which are identical or different, are each a $C_1$-$C_4$-alkyl group, $R_1$ is as defined above and the symbol ∿ is either an α-bond or a β-bond in the 6-position.

**9.** An intermediate useful in the synthesis of the compounds of formula I according to claim 1, characterized in that

it is a product of the formula

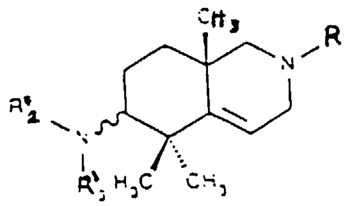

(II)

in which the symbol ∿∿∿ is an α-bond or a β-bond in the 6-position and either $R'_2$ is a hydrogen atom and $R'_3$ is a trifluoroacetyl group, or $R'_2$ and $R'_3$, which are identical or different, are each a $C_1$-$C_4$-alkyl group.

**10.** An intermediate useful in the synthesis of a compound of formula II according to claim 9, characterized in that it is a product of the formula

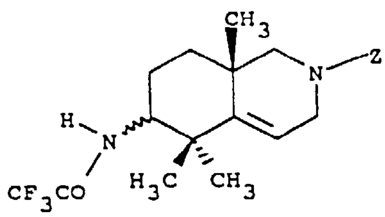

(VI)

in which the symbol ∿∿∿ is an α-bond or a β-bond in the 6-position and Z is a protective group.

**11.** A therapeutic composition, characterized in that it contains at least one compound of formula I or one of its non-toxic acid addition salts according to claim 1 in a therapeutically effective amount, in association with a physiologically acceptable excipient.

**12.** Use of a compound of formula I or one of its non-toxic acid addition salts according to claim 1 as an inhibitor of the biosynthesis of cholesterol for the preparation of a preventive or curative drug for hypercholesterolemia, atherosclerosis and mycoses.